(19) Europäisches Patentamt
European Patent Office
Office européen des brevets

(11) EP 2 700 401 B1

(12) **EUROPEAN PATENT SPECIFICATION**

(45) Date of publication and mention of the grant of the patent:
**15.01.2020 Bulletin 2020/03**

(21) Application number: **12774629.5**

(22) Date of filing: **12.04.2012**

(51) Int Cl.:
*A61K 9/70* (2006.01) *A61K 31/435* (2006.01)
*A61K 31/551* (2006.01) *A61K 47/12* (2006.01)
*A61K 47/32* (2006.01) *A61K 47/34* (2017.01)
*A61P 11/02* (2006.01) *A61P 17/04* (2006.01)
*A61P 37/08* (2006.01) *A61P 43/00* (2006.01)

(86) International application number:
**PCT/JP2012/059982**

(87) International publication number:
**WO 2012/144405 (26.10.2012 Gazette 2012/43)**

(54) **METHOD FOR PRODUCING ADHESIVE PATCH, AND ADHESIVE PATCH**

VERFAHREN ZUR HERSTELLUNG EINES KLEBEPFLASTERS UND KLEBEPFLASTER

PROCÉDÉ DE FABRICATION D'UNE PIÈCE ADHÉSIVE ET PIÈCE ADHÉSIVE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **18.04.2011 JP 2011092477**
**07.11.2011 JP 2011243189**

(43) Date of publication of application:
**26.02.2014 Bulletin 2014/09**

(73) Proprietor: **Hisamitsu Pharmaceutical Co., Inc.**
**Tosu-shi, Saga 841-0017 (JP)**

(72) Inventors:
- **HASHIMOTO Eiji**
**Tsukuba-shi**
**Ibaraki 305-0856 (JP)**
- **HAGIWARA Isao**
**Tsukuba-shi**
**Ibaraki 305-0856 (JP)**
- **NAKA Yukihisa**
**Tsukuba-shi**
**Ibaraki 305-0856 (JP)**
- **CHONO Hideharu**
**Tsukuba-shi**
**Ibaraki 305-0856 (JP)**

(74) Representative: **Grünecker Patent- und Rechtsanwälte**
**PartG mbB**
**Leopoldstraße 4**
**80802 München (DE)**

(56) References cited:
**EP-A1- 1 366 762** **EP-A1- 2 258 396**
**WO-A1-00/61120** **WO-A1-01/07018**
**WO-A1-2005/115355** **CN-A- 1 623 583**
**CN-A- 101 284 981** **KR-A- 20040 106 865**
**US-A1- 2006 110 434** **US-A1- 2008 038 328**
**US-A1- 2008 226 697** **US-A1- 2009 004 255**
**US-B1- 7 504 114**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

[Technical Field]

The present invention relates to a method for producing a patch and the patch.

[Background Art]

Various patches have been developed for many years for the purpose of improving skin permeation of drugs contained in patches. As such patches, a patch containing a drug as well as an organic acid and/or an organic acid salt has been known.

For example, Japanese Unexamined Patent Application Publication No. Hei 11-302161 (PTL 1) describes a patch containing a basic drug salt and an organic acid salt and describes that the organic acid salt is sodium acetate. International publication No. WO01/07018 (PTL 2) describes a patch containing an acid addition salt of a basic drug, an organic acid, and an organic acid salt, and describes that the organic acid includes acetic acid and lactic acid and the organic acid salt includes sodium acetate. Furthermore, International publication No. WO00/61120 (PTL 3) describes a patch containing a basic drug or a salt thereof and an organic acid or a salt thereof, and describes the organic acid includes acetic acid and propionic acid and the salt of the organic acid includes sodium acetate.

International publication No. WO2005/115355 (PTL 4) describes a patch containing a basic drug, a salt thereof, and a volatile organic acid, in which an organic acid salt or the like is further contained for the purpose of promoting the transdermal absorption of the drug, and describes that the volatile organic acid includes acetic acid and propionic acid and the organic acid salt includes sodium acetate. Furthermore, International publication No. WO02/069942 (PTL 5) describes that an organic acid is contained in an adhesive layer of a patch, and acetic acid, sodium acetate, and the like are used as the organic acid. International Application Japanese-Phase Publication No. 2004-500360 (PTL 6) describes a method for containing a hydroxide-releasing agent such as sodium acetate and potassium acetate in the presence of an aqueous fluid in a local formulation containing a drug and an inorganic hydroxide. In addition, International Publication No. WO2009/110351 (PTL 7) describes a transdermal preparation containing a drug and a complex of an organic acid and an organic acid salt, and describes that the organic acid includes acetic acid and low-molecular-weight carboxylic acid and the organic acid salt includes sodium acetate.

In PTLs 1 to 7 described above, acetic acid and/or sodium acetate and the like are described as the organic acid and/or the organic acid salt. Alkaline metal acetates such as sodium acetate, however, are generally in the form of particles having high hardness and insoluble in a non-aqueous base. Accordingly, the formation of an adhesive layer made of a non-aqueous base using such alkaline metal acetates has such problems that residual particles in the adhesive layer produce unevenness on the surface of the adhesive layer or decrease the adhesiveness of the adhesive layer; a process for pulverizing the an alkaline metal acetate is required, which makes a production process complicated; and the obtained patch is not able to attain a sufficient effect of improving the skin permeation because it is difficult to uniformly disperse an alkaline metal acetate in the adhesive layer.

Furthermore, the formation of the adhesive layer using acetic acid has such problems that it is difficult to obtain a patch containing a sufficient amount of acetic acid to produce a target effect of improving the skin permeation of the drug because the volatility of acetic acid is high, or the content of acetic acid decreases during the use or the storage of the patch and accordingly the effect of improving the skin permeation of the drug decreases.

In addition, a patch in which the skin permeation of a drug is attempted to increase by making the drug itself into a free form (free type) instead of a salt form has been also conventionally known.

For example, Japanese Unexamined Patent Application Publication Nos. Hei 3-83924 (PTL 8), Hei 7-33665 (PTL 9), and Hei 8-193030 (PTL 10) describe patches using a free type drug (emedastine). Furthermore, as methods for making basic drugs contained in patches into a free type, Japanese Unexamined Patent Application Publication No. Hei 2-255612 (PTL 11) describes a method for setting the pH of an adhesive layer containing a basic drug to 7 or more, and Japanese Unexamined Patent Application Publication No. Hei 3-197420 (PTL 12) describes a method for containing a supplemental acid such as acetic acid in the salt of a drug. However, the free type drugs as described in PTLs 8 to 12 have problems of poor storage stability as compared with drugs in the salt form and coloration of the adhesive layer during the storage of the patch.

US 2008/0226697 A describes a pressure-sensitive adhesive composition for transdermal absorption which comprises a drug, a transdermal absorption promoter for the drug and polyvinylpyrrolidone for stabilizing the transdermal absorption promoter.

US 2009/0004255 A describes a patch which contains a volatile organic acid and a basic drug which forms an ion pair with an anion component.

US 2006/0110434 A discloses a patch containing a basic drug, a melting point lowering agent such acetic acid and/or sodium acetate and an adhesive base.

[Citation List]

[Patent Literature]

[PTL 1] Japanese Unexamined Patent Application Publication No. Hei 11-302161
[PTL 2] International publication No. WO01/07018
[PTL 3] International publication No. WO00/61120
[PTL 4] International publication No. WO2005/115355
[PTL 5] International publication No. WO02/069942
[PTL 6] International Application Japanese-Phase Publication No. 2004-500360
[PTL 7] International publication No.WO2009/110351
[PTL 8] Japanese Unexamined Patent Application Publication No. Hei 3-83924
[PTL 9] Japanese Unexamined Patent Application Publication No. Hei 7-33665
[PTL 10] Japanese Unexamined Patent Application Publication No. Hei 8-193030
[PTL 11] Japanese Unexamined Patent Application Publication No. Hei 2-255612
[PTL 12] Japanese Unexamined Patent Application Publication No. Hei 3-197420


[Summary of Invention]

[Technical Problem]

The present invention has been made in consideration of the above-described problems in the conventional techniques. An object of the present invention is to provide a method for producing a patch, which can easily give the patch excellent in skin permeation of a drug and can reduce variation in skin permeation of the drug for each pharmaceutical preparation, and the patch obtained by the production method.

[Solution to Problem]

The present inventors have earnestly studied in order to achieve the above object. As a result, the present inventors have found that, in a method for producing a patch including a support layer and an adhesive layer, the formation of the adhesive layer using an adhesive layer composition obtained by mixing a drug and a nonaqueous adhesive base with an alkali metal diacetate in a specific ratio allows the alkali metal diacetate to significantly improve the skin permeation of the drug in the obtained patch. The present inventors have completed the present invention by finding that such a method for producing a patch enables easy production of the patch because a special process such as pulverization of particles is not necessary, and further can reduce variation in skin permeation of the drug for each pharmaceutical preparation obtained.

The method for producing a patch of the present invention is a method for producing a patch including a support layer and an adhesive layer, comprising the step of forming the adhesive layer with use of an adhesive layer composition obtained by mixing an alkali metal diacetate, a drug, and a nonaqueous adhesive base such that the molar ratio between the drug and the alkali metal diacetate (the number of moles of the drug : the number of moles of the alkali metal diacetate) is from 1:0.5 to 1:15, wherein the drug is at least one selected from the group consisting of fumaric acid addition salts of a basic drug, maleic acid addition salts of a basic drug, citric acid addition salts of a basic drug, and hydrochloric acid addition salts of a basic drug,
the basic drug is at least one selected from the group consisting of emedastine, setiptiline, and oxybutynin, and
the nonaqueous adhesive base is at least one selected from the group consisting of a styrene-isoprene-styrene block copolymer, a (meth)acrylate (co)polymer, polyisobutylene, and a silicone polymer.

In the method for producing a patch of the present invention, the alkali metal diacetate is preferably sodium diacetate.

In the method for producing a patch of the present invention, the drug is preferably an acid addition salt of a basic drug, more preferably a polybasic acid addition salt or hydrochloric acid addition salt of a basic drug, and still more preferably at least one selected from the group consisting of fumaric acid addition salts of a basic drug, maleic acid addition salts of a basic drug, citric acid addition salts of a basic drug, and hydrochloric acid addition salts of a basic drug. Moreover, the basic drug is preferably at least one selected from the group consisting of emedastine, setiptiline, and oxybutynin.

In the method for producing a patch of the present invention, the nonaqueous adhesive base is preferably at least one selected from the group consisting of a styrene-isoprene-styrene block copolymer, a (meth)acrylate (co)polymer,

polyisobutylene, and a silicone polymer.

The patch of the present invention is a patch comprising a support layer and an adhesive layer, in which the adhesive layer is formed with use of an adhesive layer composition obtained by mixing an alkali metal diacetate, a drug, and a nonaqueous adhesive base such that the molar ratio between the drug and the alkali metal diacetate (the number of moles of the drug : the number of moles of the alkali metal diacetate) is from 1:0.5 to 1:15.

The reason to achieve the above object is not always clear, but the present inventors suppose that the reason is as follows. That is, in the production method of the present invention, the alkali metal diacetate according to the present invention can be contained in the adhesive layer of the patch as a skin permeation enhancer for the drug when the adhesive layer is prepared, based on the finding found by the present inventors that, even without using acetic acid and/or an alkaline metal salt of acetic acid which are conventionally used, the use of the alkali metal diacetate in a specific ratio in combination with the drug surprisingly provides an excellent effect of improving the skin permeation of the drug as compared with the case using the acetic acid and/or the alkaline metal salt of the acetic acid. The powder particles of the alkali metal diacetate have lower hardness than the powder particles of the alkaline metal acetate and can be uniformly dispersed in the adhesive layer without requiring a special process such as pulverization of the particles even if used together with the nonaqueous adhesive base. The production method of the present invention easily provides the patch in which the alkali metal diacetate sufficiently exhibits an effect of improving the skin permeation of the drug. Furthermore, the present inventors suppose that it is possible to obtain the patch that has good appearance without unevenness on the surface of the adhesive layer and has decrease in adhesiveness suppressed.

Since the alkali metal diacetate does not have volatility unlike acetic acid, the production method of the present invention can provide the patch that always contains a certain amount of alkali metal diacetate and can exhibit a certain level of the effect of improving the skin permeation. Accordingly, the present inventors suppose that the variation in skin permeability of the drug for each pharmaceutical preparation can be reduced. Furthermore, since the decreased amount of the alkali metal diacetate is smaller than that of the acetic acid during the use or the storage of the obtained patch, the present inventors suppose that the decrease in effect of improving the skin permeation of the drug can be suppressed. When a solvent is used in the production of the patch, the use of a compound having large polarity such as the acetic acid with the solvent tends to cause phase separation and the solvent to be used is limited. However, there is no such limitation on the alkali metal diacetate according to the present invention, and the present inventors suppose that the production of the patch becomes easier.

Furthermore, since the patch obtained by the production method of the present invention attains a sufficient effect of improving the skin permeation of the drug even without dependent on a method using a free type drug which has beer, conventionally conducted for the purpose of improving the skin permeability of the drug contained in a patch, the present inventors suppose that a more stable drug in the salt form can be used, making it possible to further improve the storage stability.

[Advantageous Effects of Invention]

The present invention can provide the method for producing a patch, which can easily give the patch excellent in skin permeation of the drug and can reduce variation in skin permeation of the drug for each pharmaceutical preparation, and the patch obtained by the production method.

[Brief Description of Drawings]

[Fig. 1] Fig. 1 is a graph showing a spectrum of sodium diacetate at an X-ray diffraction angle of 2θ°.
[Fig. 2] Fig. 2 is a graph showing a spectrum of sodium acetate at an X-ray diffraction angle of 2θ°.
[Fig. 3] Fig. 3 is a graph showing the results of a skin permeation test for the patches obtained in Example 1 and Comparative Example 1.
[Fig. 4] Fig. 4 is a graph showing the results of a skin permeation test for the patches obtained in Example 2 and Comparative Example 2.
[Fig. 5] Fig. 5 is a graph showing the results of a skin permeation test for the patches obtained in Example 3 and Comparative Example 3.
[Fig. 6] Fig. 6 is a graph showing the results of a skin permeation test for the patches obtained in Example 5 and Comparative Example 5.
[Fig. 7] Fig. 7 is a graph showing a spectrum of the patch obtained in Example 3 at an X-ray diffraction angle of 2θ°.
[Fig. 8] Fig. 8 is a graph showing a spectrum of the patch obtained in Comparative Example 3 at an X-ray diffraction angle of 2θ°.
[Fig. 9] Fig. 9 is a graph showing a spectrum of the patch obtained in Example 4 at an X-ray diffraction angle of 2θ°.

[Fig. 10] Fig. 10 is a graph showing a spectrum of the patch obtained in Comparative Example 4 at an X-ray diffraction angle of 2θ°.

[Fig. 11] Fig. 11 is a graph showing the results of a storage stability evaluation test for the patches obtained in Example 1 and Comparative Example 1.

[Description of Embodiments]

The present invention will be described below in detail with reference to its preferred embodiments.

The method for producing a patch of the present invention is a method for producing a patch including a support layer and an adhesive layer, characterized by comprising the step of forming the adhesive layer with use of an adhesive layer composition obtained by mixing an alkali metal diacetate, a drug, and a nonaqueous adhesive base.

(Alkali Metal Diacetate)

The alkali metal diacetate according to the present invention is a complex compound in which two acetic acid molecules and one alkali metal atom form a salt, expressed by the following general formula (1) :

$$MH(CH_3COO)_2 \ldots \qquad (1)$$

wherein, M represents an alkali metal atom. When having water of crystallization, the complex compound is expressed by the following general formula (2) :

$$CH_3COOM \cdot CH_3COOH \cdot XH_2O \ldots \qquad (2)$$

wherein, M represents an alkali metal, and X represents an integer. The alkali metal diacetate according to the present invention is preferably an anhydride.

The alkali metal diacetate is preferably in the form of powder particles, and the particle size of the powder particle is preferably 150 μm or less and more preferably 3 to 10 μm. When the particle size is less than the lower limit, the powder of the alkali metal diacetate tends to absorb moisture, so that the adhesive layer contains water; when it is over the upper limit, however, the effect of improving the skin permeability of the drug tends to decrease.

The alkali metal atoms include lithium (Li), sodium (Na), potassium (K), rubidium (Rb), cesium (Cs), and francium (Fr). As the alkali metal diacetate according to the present invention, one alkali metal diacetate may be used alone, or two or more alkali metal diacetates may be used in combination, but sodium diacetate ($NaH(CH_3COO)_2$) is preferred with the view of easy availability.

Sodium diacetate will be described below as an example of the alkali metal diacetate according to the present invention. Sodium diacetate is in the form of crystalline powder particles having a melting point of 323°C to 329°C, and can be obtained, for example, by mixing acetic acid and sodium acetate in water in a molar ratio (the number of moles of acetic acid : the number of moles of sodium acetate) of 1 : 1 and then removing water for crystallization. Since acetic acid and sodium acetate are soluble in water, mixing both in water in this way can provide the sodium diacetate according to the present invention. Although acetic acid is soluble in nonaqueous systems such as organic solvents and the nonaqueous adhesive base described below, sodium acetate has extremely low solubility therein. Accordingly, it is difficult to obtain a sufficient amount of sodium diacetate even if acetic acid and sodium acetate is simply mixed in a molar ratio (the number of moles of acetic acid : the number of moles of sodium acetate) of 1 : 1, and large amounts of unreacted acetic acid and sodium acetate tend to remain.

In the production method of the present invention, the formation of the adhesive layer using the adhesive layer composition obtained by mixing the alkali metal diacetate according to the present invention with the drug and the nonaqueous adhesive base described below can improve the skin permeability of the drug in the obtained patch. Accordingly, the alkali metal diacetate according to the present invention can be used as a skin permeation enhancer for the drug.

(Drug)

For the drug according to the present invention, there is no particular limitation on drug effect, and one drug may be used alone, or two or more drugs may be used in combination. The drug is preferably a drug (basic drug) having a basic functional group such as an amino group with the view of excellent skin permeability. The basic drugs include hypnotics and sedatives (flurazepam, rilmazafone, medetomidine, dexmedetomidine), stimulants and psychostimulants (methamphetamine, methylphenidate), psychoneurotic agents (imipramine, diazepam, sertraline, fluvoxamine, paroxetine, citalopram, fluoxetine, alprazolam, haloperidol, clomipramine, amitriptyline, desipramine, amoxapine, maprotiline,

mirtazapine, setiptiline, duloxetine, diazepam, etizolam), local anesthetics (lidocaine, procaine, tetracaine, dibucaine), agents for urinary organs (oxybutynin, tamuslosin, propiverine, imidafenacin, solifenacin, darifenacin, tolterodine), skeletal muscle relaxants (tizanidine, eperisone, pridinol, suxamethonium), agents for genital organs (ritodrine, meluadrine), agents for autonomic nerves (carpronium, neostigmine, bethanechol), anti-Parkinson's disease agents (pergolide, bromocriptine, trihexyphenidyl, amantadine, ropinirole, talipexole, pramipexole, rotigotine, cabergoline, selegiline, rasagiline), antimigraine agents (dihydroergotamine, sumatriptan, ergotamine, flunarizine, cyproheptadine), antihistamines (clemastine, diphenhydramine, chlorpheniramine, diphenylpyraline), bronchodilators (tulobuterol, procaterol, salbutamol, clenbuterol, fenoterol, terbutaline, isoprenaline), a cardiotonic (isoprenaline), peripheral vasodilators (nicametate, tolazoline), stop-smoking aids (nicotine, varenicline), agents for circulation organs (atenolol, bisoprolol, metoprolol, carvedilol, carteolol, valsartan, clonidine), antiarrhythmic agents (propranolol, alprenolol, procainamide, mexiletine), antiulcer agents (proglumide, cetraxate, spizofurone, cimetidine), prokinetic agents (domperidone, cisapride), antiallergic agents (ketotifen, azelastine, emedastine), an antivirotic (aciclovir), anti-Alzheimer agents (donepezil, tacrine, arecoline, galanthamine, rivastigmine), serotonin receptor antagonist antiemetics (ondansetron, granisetron, ramosetron, azasetron), analgesics (morphine, codeine, fentanyl, oxycodone), and antifungal agents (terbinafine, butenafine, amorolfine, neticonazole, miconazole, luliconazole, itraconazole). One of these may be used alone, or two or more of these may be used in combination. The basic drug is preferably at least one selected from the group consisting of emedastine, setiptiline, and oxybutynin with the view of more excellent skin permeability.

The drug according to the present invention is more preferably an pharmaceutically acceptable acid addition salt of the basic drug with the view to obtain excellent storage stability of the drug and suppress discoloration of the adhesive layer due to decomposition of the drug and with the view to suppress irritation to the skin. The patch of the present invention acquires excellent skin permeation even if such a drug in the salt form is used as the drug. The acids include monobasic acids such as hydrochloric acid, hydrobromic acid, and methanesulfonic acid; and polybasic acids such as fumaric acid, maleic acid, citric acid, and tartaric acid. Among these, polybasic acids such as maleic acid, fumaric acid, citric acid, and tartaric acid, or hydrochloric acid is preferred with the view of excellent skin permeation of the drug.

Examples of fumaric acid addition salts of the basic drug include emedastine fumarate, clemastine fumarate, formoterol fumarate, and quetiapine fumarate. In addition, examples of maleic acid addition salts of the basic drug include setiptiline maleate, chlorpheniramine maleate, elanaprilmaleate, methylergometrine maleate, trimebutine maleate, irsogladine maleate, timolol maleate, carpipramine maleate, fluvoxamine maleate, trifluoperazine maleate, levomepromazine maleate, enalapril maleate, and fluphenazine maleate.

Examples of citric acid addition salts of the basic drug include fentanyl citrate, pentoxyverine citrate, tamoxifen citrate, clomifene citrate, diethylcarbamazine citrate, tandospirone citrate, toremifene citrate, and sildenafil citrate. Examples of tartaric acid addition salts of the basic drug include ifenprodil tartrate, metoprolol tartrate, alimemazine tartrate, butorphanol tartrate, varenicline tartrate, tolterodinetartrate, and zolpidem tartrate.

Examples of hydrochloric acid addition salts of the basic drug include oxybutynin hydrochloride, lofepramine hydrochloride, maprotiline hydrochloride, a perospirone hydrochloride hydrate, trihexyphenidyl hydrochloride, biperiden hydrochloride, azelastine hydrochloride, nortriptyline hydrochloride, imipramine hydrochloride, baclofen hydrochloride, diphenylpyraline hydrochloride, cloperastine hydrochloride, epinastine hydrochloride, cyclobenzaprine hydrochloride, talipexole hydrochloride, cyproheptadine hydrochloride, mianserin hydrochloride, pilocarpine hydrochloride, ambroxol hydrochloride, a cevimeline hydrochloride hydrate, lomerizine hydrochloride, verapamil hydrochloride, guanfacine hydrochloride, triprolidine hydrochloride, loperamide hydrochloride, benazepril hydrochloride, prazosin hydrochloride, isoprenaline hydrochloride, promethazine hydrochloride, dicyclomine hydrochloride, dexmedetomidine hydrochloride, ramosetron hydrochloride, alosetron hydrochloride, mianserin hydrochloride, perospirone hydrochloride, carteolol hydrochloride, and tulobuterol hydrochloride.

The drug according to the present invention is more preferably at least one selected from the group consisting of the fumaric acid addition salts of the basic drug, the maleic acid addition salts of the basic drug, the citric acid addition salts of the basic drug, and the hydrochloric acid addition salts of the basic drug, and still more preferably at least one selected from the group consisting of the fumaric acid addition salts of the basic drug, the maleic acid addition salts of the basic drug, and the hydrochloric acid addition salts of the basic drug, with the view to obtain the patch excellent in storage stability and skin permeation of the drug. Furthermore, the drug according to the present invention is particularly preferably at least one selected from the group consisting of emedastine fumarate, setiptiline maleate, and oxybutynin hydrochloride with the view to obtain the patch more excellent in storage stability and skin permeation of the drug.

As examples of the drug according to the present invention, emedastine fumarate, setiptiline maleate, and oxybutynin hydrochloride will be described below.

The emedastine fumarate is a drug expressed by the following formula (3).

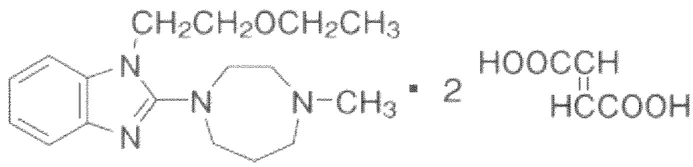

(3)

The emedastine fumarate (1-(2-ethoxyethyl)-2-(hexahydro-4-methyl-1H-1,4-diazep in-1-yl) benzimidazole difumarate) is a benzimidazole derivative found by screening targeted for the antiallergic action. This emedastine fumarate is confiemed to have an antiallergic action, an antihistaminic action, an inhibitory action on histamine release, and the like. As pharmaceutical preparations using such emedastine fumarate, for example, capsules effective against allergic rhinitis, urticaria, and the like are known. In the present invention, such emedastine fumarate is preferably used with the view that there is a tendency to obtain the patch excellent in storage stability of the drug and prevent the patch from being colored with time.

The setiptiline maleate is a drug expressed by the following formula (4).

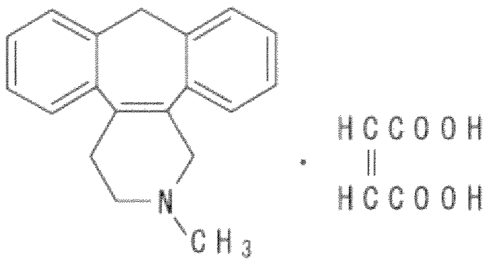

(4)

The setiptiline maleate (2,3,4,9-tetrahydro-2-methyl-1H-dibenzo[3,4,6,7]cycloh epta[1,2-c] pyridine maleate) is a tetracyclic antidepressant that improves neurotransmission in the brain. In the present invention, such setiptiline maleate is preferably used with the view that there is a tendency to obtain the patch excellent in storage stability of the drug and prevent the patch from being colored with time.

The oxybutynin hydrochloride is a drug expressed by the following formula (5).

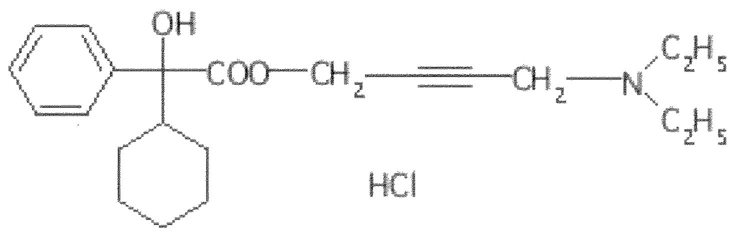

(5)

The oxybutynin hydrochloride (4-diethylamino-2-butynyl-α-cyclohexyl-α-phenylglycoll ate hydrochloride) is an anticholinergic drug that increases bladder capacity by suppressing bladder contraction. In the present invention, the oxybutynin hydrochloride is preferably used with the view that there is a tendency to obtain the patch excellent in storage stability of the drug and prevent the patch from being colored with time.

(Nonaqueous Adhesive Base)

The nonaqueous adhesive base according to the present invention is a base that mainly exhibits pressure-sensitive adhesion in the adhesive layer of the patch, and is substantially free of water. The expression "substantially free of water" here means that water is not intentionally blended and the content of water obtained by the measurement with the Karl Fischer method based on the Japanese pharmacopoeia is less than 10% in the adhesive layer.

Examples of the nonaqueous adhesive bases according to the present invention include a (meth)acrylate (co)polymer, a rubber-based adhesive, a silicone polymer, and a polyurethane-based adhesive. One of these may be used alone, or two or more of these may be used in combination.

The (meth)acrylate (co)polymer is a (co)polymer in which acrylate and/or methacrylate are polymerized as main monomer units, or copolymerized with any submonomer if necessary. Examples of the main monomer units include

methyl (meth)acrylate, ethyl (meth)acrylate, butyl (meth)acrylate, hexyl (meth)acrylate, heptyl (meth)acrylate, octyl (meth)acrylate, and 2-ethylhexyl (meth) acrylate. Although one of these may be used alone, or two or more of these may be used in combination, 2-ethylhexyl (meth)acrylate is preferably used with the view to obtain the patch excellent in adhesion. Examples of the submonomer include, but are not particularly limited to, N-vinyl-2-pyrrolidone, methylvinyl pyrrolidone, (meth)acrylic acid, 2-ethylhexyl (meth)acrylate, and vinyl acetate.

Examples of the rubber-based adhesive include natural rubber, polyisobutylene, polyvinyl ether, polyisoprene, polybutadiene, a styrene-butadiene copolymer, a styrene-isoprene copolymer, and a styrene-isoprene-styrene block copolymer. One of these may be used alone, or two or more of these may be used in combination.

The nonaqueous adhesive base according to the present invention is preferably at least one selected from the group consisting of a styrene-isoprene-styrene block copolymer, a (meth)acrylate (co)polymer, polyisobutylene, and a silicone polymer with the view to obtain the patch excellent in skin permeation of the drug and adhesion.

(Adhesive Layer Composition)

In the production method of the present invention, the alkali metal diacetate, the drug, and the nonaqueous adhesive base are mixed to obtain an adhesive layer composition.

In the adhesive layer composition according to the present invention, the molar ratio between the drug and the alkali metal diacetate (the number of moles of the drug : the number of moles of the alkali metal diacetate) needs to be from 1:0.5 to 1:15. In the present invention, the skin permeation of the drug in the patch can be relatively increased as the number of moles of the alkali metal diacetate is increased with respect to the number of moles of the drug. When the molar ratio of the alkali metal diacetate to the drug is less than the lower limit, the effect of improving the skin permeation of the drug is not sufficiently exhibited; when it is over the upper limit, however, it is difficult to uniformly mix the alkali metal diacetate in the adhesive layer, which decreases the cohesiveness of the adhesive layer.

The content of the alkali metal diacetate is preferably 1% to 18% by mass, and more preferably 3% to 12% by mass in the obtained adhesive layer. When the content of the alkali metal diacetate is less than the lower limit, the effect of improving the skin permeation of the drug tends to decrease; when it is over the upper limit, however, there is a tendency that the adhesion of the patch decreases or the thickness of the adhesive layer is hardly adjusted to be even.

Although the content of the drug can be appropriately adjusted according to the employed drug and the intended drug effect in the adhesive layer composition according to the present invention, it is usually preferred that the content of the drug in the obtained adhesive layer is 1% to 50% by mass. For example, when the drug according to the present invention is the emedastine fumarate and/or the setiptiline maleate, regarding the content of these, the content in the obtained adhesive layer is preferably 1% to 15% by mass. When the drug according to the present invention is the oxybutynin hydrochloride, regarding the content thereof, the content in the obtained adhesive layer is preferably 1% to 15% by mass. When the content of the drug is less than the lower limit, the effect as a drug tends not be sufficiently exhibited in the patch; when the content of the drug is over the upper limit, however, the adhesion of the patch tends to decrease.

In the adhesive layer composition according to the present invention, the content of the nonaqueous adhesive base is not particularly limited and can be adjusted according to the contents of the alkali metal diacetate and the drug.

The adhesive layer composition according to the present invention may further contain a component other than the alkali metal diacetate, the drug, and the nonaqueous adhesive base if necessary without inhibiting the effects of the present invention. Examples of the component include tackifier resins, plasticizers, transdermal absorption promoting agents, solubilizers, stabilizers, and fillers. When such a component is contained, the content thereof is preferably 85% by mass or less in the obtained adhesive layer.

Since pulverization of the powder particles of the alkali metal diacetate is not necessary for the adhesive layer composition according to the present invention, a method for mixing the adhesive layer composition is not particularly limited, but preferably a method capable of uniformly mixing mixing the adhesive layer composition. Examples thereof include mixing methods with a propeller mixer, a paddle mixer, an anchor mixer, a planetary mixer, and a grinding machine.

(Formation of Adhesive Layer)

The production method of the present invention is characterized by comprising the step of forming the adhesive layer using the adhesive layer composition. The adhesive layer is preferably formed on one surface of the support layer.

The support layer according to the present invention is not particularly limited and those known as a support layer of the patch can be appropriately employed. Examples of materials for such a support layer include synthetic resins such as polyethylene, polypropylene, polybutadiene, an ethylene-vinylacetate copolymer, a vinyl acetate-vinyl chloride copolymer, polyvinyl chloride, polyamide, polyester, nylon, cellulose derivatives, and polyurethane. Forms of the support layer include a film; a sheet; a sheet-like porous body; a sheet-like foam; textiles such as a woven fabric, a knitted fabric, and a nonwoven fabric; and a laminate of these. The thickness of the support layer is not particularly limited, but it is

usually preferred that the thickness is about 2 to 3000 μm.

The method for forming the adhesive layer is not particularly limited except that water is not blended and a known method for forming an adhesive layer can be appropriately employed. Examples thereof include a solvent method and a hot melt method.

In the solvent method, the adhesive layer composition dissolved and/or dispersed in a solvent is first coated on one surface of the support layer at a desired thickness, and then the layer on which this adhesive layer composition is coated is heated to remove the solvent, thereby forming the adhesive layer according to the present invention. The thickness of the coating is not particularly limited, but it is usually preferred that the thickness of the obtained adhesive layer is about 10 to 300 μm. The conditions for the heating can be appropriately selected according to the solvent, but it is preferred that the temperature condition is usually 60°C to 120°C and it is preferred that the heating condition is usually for 2 to 30 minutes.

The solvent is preferably a nonaqueous organic solvent. The nonaqueous organic solvent can be appropriately selected according to the kinds of the alkali metal diacetate, the drug, the nonaqueous adhesive base, and the like to be used. Examples thereof include lower alcohols such as methanol, ethanol, and isopropanol; toluene, xylene, pentane, n-hexane, cyclohexane, heptane, octane, methyl acetate, ethyl acetate, propyl acetate, methyl butyrate, ethyl butyrate, and propyl butyrate. When the adhesive layer composition according to the present invention is dissolved and/or dispersed in the solvent, the concentration of the nonvolatile content of the adhesive layer composition is preferably 10% to 70% by mass. When the concentration is less than the lower limit, the energy efficiency associated with solvent drying in a production facility tends to decrease; when the concentration is over the upper limit, however, there is a tendency that the adhesive layer composition is hardly coated on the support layer or a release liner layer when forming the adhesive layer.

In the hot melt method, the adhesive layer composition is first thermally melted while mixing, this is coated at a desired thickness on one surface of the support layer, and this is then cooled at room temperature to form the adhesive layer according to the present invention. The thickness of the coating is as described in the solvent method. The condition of the thermal melting can be appropriately selected according to the composition of the adhesive layer composition, but it is preferred that the condition is usually 70°C to 200°C. In the production method of the present invention, a patch sheet thus obtained including the support layer and the adhesive layer is appropriately cut to provide the patch according to the present invention.

The production method of the present invention preferably comprises the step of further laminating a release liner layer on the surface of the adhesive layer opposite to the support layer. The release liner layer is not particularly limited and those known as a release liner layer of the patch can be appropriately employed. Examples of such a release liner layer include polyester, polypropylene, polyethylene, a paper, or a film made of a laminate of these, and those processed with a mold release treatment such as silicone coating are preferred to acquire easy releasing. The thickness of the release liner layer is not particularly limited, but it is preferred that the thickness is usually about 2 to 3000 μm. When the release liner layer is laminated in the production method of the present invention, the adhesive layer composition may be first coated on one surface of the release liner layer to form the adhesive layer in the step of forming the adhesive layer, and the support layer may be then laminated on the surface of the adhesive layer opposite to the release liner layer.

The production method of the present invention can provide the patch comprising the support layer and the adhesive layer easily and reproducibly, characterized in that the adhesive layer contains the nonaqueous adhesive base, the drug, and the alkali metal diacetate, and the molar ratio between the drug and the alkali metal diacetate (the number of moles of the drug : the number of moles of the alkali metal diacetate) is from 1:0.5 to 1:15 in the adhesive layer. Such a patch has excellent skin permeation of the drug and small variation in skin permeation of the drug for each pharmaceutical preparation.

[Examples]

Although the present invention will be described below in more detail based on Examples and Comparative Examples, the present invention is not limited to the following Examples. In each Example and Comparative Example, the skin permeation test, the measurement of X-ray diffraction angle, and the storage stability evaluation test were each performed by the following procedures.

(Skin Permeation Test)

First, fat on the dermis side of the skin excised from the back of 6 to 8-month-old Clawn miniature pigs was removed carefully, and the skin was installed in a flow-through cell such that the dermis side was on the receptor tank side. Next, a patch that had been obtained by cutting into a size of 3 $cm^2$ and removing a release liner was attached to the corneum side of this skin. Water at 32°C was circulated at the periphery of the receptor tank of the flow-through cell, and physiological saline (32°C) was introduced into the receptor tank at a flow rate of about 3 ml per hour. A sample

liquid was taken from the receptor tank every 6 hours for 24 hours, and the concentration of the drug in the receptor tank was quantitated by a high-performance liquid chromatographic method for each sample liquid taken. The cumulative amount of the drug permeated [Q] was calculated by the following equation:

$$\text{Cumulative Amount of Drug Permeated [Q] } (\mu g/cm^2) = [\text{Concentration of Drug } (\mu g/ml) \times \text{Flow Rate (ml)}] / \text{Area of Adhesive Patch } (cm^2).$$

Pharmaceutical preparations having a large cumulative amount of the drug permeated is recognized as ones excellent in skin permeation.

(Measurement of X-ray Diffraction Angle)

First, X-ray diffraction angles of sodium diacetate and sodium acetate were measured as reference samples. Depressions on a glass plate for the measurement were each filled with sodium diacetate or sodium acetate to make a measuring surface even and then the X-ray diffraction angles were measured with the following instrument under the following measurement conditions:

Instrument: X' Pert-PRO MPD (produced by PANalytical B.V.)
X-ray: CuK$\alpha$
Scan angle: 5° to 50°
Step size: 0.0167°
Time per step: 10.160 seconds.

The spectrum of sodium diacetate at an X-ray diffraction angle of 2$\theta$° is shown in Fig. 1. In the result shown in Fig. 1, a peak from sodium diacetate was observed near 22.4°. The spectrum of sodium acetate at an X-ray diffraction angle of 2$\theta$° is shown in Fig. 2. In the result shown in Fig. 2, a peak from sodium acetate was observed near 8.7°.
Next, the X-ray diffraction angles of the patches obtained in respective Examples and Comparative Examples were measured. A measurement sample was made by fixing the support layer side of the patch to a nonreflective plate with a double-sided adhesive tape and removing the release liner to expose the adhesive layer, and measured with the above instrument under the above measurement conditions.

(Storage Stability Evaluation Test)

The patch was packaged in an aluminum laminated plastic packing material and hermetically sealed with a heat sealer. It was allowed to stand and stored at 80°C for one week. The drugs were extracted from the adhesive layer of the patch before the storage and after one-week storage, respectively to make sample liquids. The concentration of the drug in the adhesive layer was quantitated by a high-performance liquid chromatographic method for each sample liquid taken. The residual percentage (%) of the drug was calculated on the basis of the concentration of the drug (% by mass) before the storage. The appearances of the patch before the storage and after one-week storage were visually observed, respectively.

(Example 1)

First, 10.0 parts by mass of sodium diacetate, 5.0 parts by mass of emedastine fumarate, 18.0 parts by mass of a styrene-isoprene-styrene block copolymer (SIS), 5.0 parts by mass of a methacrylate copolymer (trade name: EUDRAGIT, produced by Rohm Pharma GmbH), 8.0 parts by mass of polyisobutylene (PIB), 39.0 parts by mass of a petroleum-based tackifier resin (trade name: Alcon, produced by Arakawa Chemical Industries, Ltd.), 5.0 parts by mass of sucrose fatty acid ester, 5.0 parts by mass of sorbitan trioleate, and 5.0 parts by mass of diisopropanolamine were mixed in toluene and stirred with a propeller mixer to obtain an uniform adhesive layer composition (the concentration of a nonvolatile content: 50% by mass). Next, this adhesive layer composition was coated on one surface of a 75-$\mu$m-thick mold release liner layer made of polyethylene terephthalate such that the thickness after drying was 100 $\mu$m. This was dried at 60°C for 20 minutes to form an adhesive layer. Next, a 30-$\mu$m-thick polyethylene terephthalate film was laminated as the support layer on the surface of the adhesive layer opposite to the release liner layer to provide a patch. The composition (except for toluene) of the adhesive layer composition is shown in Table 1. In the adhesive layer of the

obtained patch, the molar ratio between emedastine fumarate and sodium diacetate (the number of moles of emedastine fumarate : the number of moles of sodium diacetate) was 1:7.5.

(Examples 2 to 4)

Patches were obtained in the same way as in Example 1 except that the composition of an adhesive layer composition was as shown in Table 1. In an adhesive layer of the obtained patch, the molar ratios of emedastine fumarate to sodium diacetate (the number of moles of emedastine fumarate : the number of moles of sodium diacetate) were 1:7.5 in Example 2 and 1:6 in Example 3. The molar ratio between setiptiline maleate and sodium diacetate (the number of moles of setiptiline maleate : the number of moles of sodium diacetate) in Example 4 was 1:10.5.

(Comparative Examples 1 to 4)

Each patch was obtained in the same way as in Example 1 except that the composition of an adhesive layer composition was as shown in Table 1.

[Table 1]

| Composition (parts by weight) | Example 1 | Example 2 | Example 3 | Comparative Example 1 | Comparative Example 2 | Comparative Example 3 | Example 4 | Comparative Example 4 |
|---|---|---|---|---|---|---|---|---|
| Sodium Diacetate | 10.0 | 10.0 | 8.0 | - | - | - | 9.9 | - |
| Sodium Acetate | - | - | - | - | - | 4. 6 | - | 5.7 |
| Acetic Acid | - | - | - | - | - | 3.4 | - | 4.2 |
| Diethyl Sebacate | - | 10.0 | 10.0 | - | 10.0 | 10.0 | 5.0 | 5.0 |
| Glycerol | - | - | 1.5 | - | - | 1.5 | - | - |
| Oleic Acid | - | - | - | - | 10.0 | - | - | - |
| Peru Balsam | - | 5.0 | - | - | 5.0 | - | - | - |
| Sucrose Fatty Acid Ester | 5.0 | - | - | 5.0 | - | - | - | - |
| Sorbitan Trioleate | 5.0 | - | - | 5.0 | - | - | - | - |
| Diisopropanolamine | 5.0 | - | - | 5.0 | - | - | - | - |
| Liquid Paraffin | - | - | 5.0 | - | - | 5.0 | 9.9 | 9.9 |
| CMC (Carboxymethylcellulose)-Na | - | 5.0 | - | - | 5.0 | - | - | - |
| Methacrylate Copolymer | 5.0 | 5.0 | - | 5.0 | 5.0 | - | - | - |
| Petroleum-Based Tackifier Resin | 39.0 | 36.0 | 42.3 | 43.0 | 36.0 | 42.3 | 3 1 .6 | 31.6 |
| SIS | 18.0 | 16.8 | 19.7 | 22.0 | 16.8 | 19.7 | 26.4 | 26.4 |
| PIB | 8.0 | 7.2 | 8.5 | 10.0 | 7.2 | 8.5 | 12.3 | 12.3 |
| Dibutylhydroxy-toluene (BHT) | - | - | - | - | - | - | 2.5 | 2.5 |
| Emedastine Fumarate | 5.0 | 5.0 | 5.0 | - | 5.0 | 5.0 | - | - |
| Emedastine (Free Type) | - | - | - | 5.0 | - | - | - | - |
| Setiptiline Maleate | - | - | - | - | - | - | 2.5 | 2.5 |
| Total | 100.0 | 100.0 | 100.0 | 100.0 | 100.0 | 100.0 | 100.1 | 100.1 |

(Example 5, Comparative Example 5)

Each patch was obtained in the same way as in Example 1 except that the composition of an adhesive layer composition is as shown in the following Table 2. In the adhesive layer of the patch obtained in Example 5, the molar ratio between oxybutynin hydrochloride and sodium diacetate (the number of moles of oxybutynin hydrochloride : the number of moles of sodium diacetate) was 1:5.

[Table 2]

| Composition (parts by weight) | Example 5 | Comparative Example 5 |
|---|---|---|
| Sodium Diacetate | 18.0 | - |
| Sodium Acetate | - | 9.0 |
| Citric Acid | - | 2.5 |
| Liquid Paraffin | 20.0 | 10.5 |
| Petroleum-Based Tackifier Resin | 34.7 | 32.0 |
| SIS | 17.3 | 18.0 |
| PIB | - | 13.0 |
| Oxybutynin Hydrochloride | 10.0 | 15.0 |
| Total | 100.0 | 100.0 |

The patches obtained in Examples 1 to 5 and Comparative Examples 1 to 5 were each subjected to the skin permeation test. The results of Example 1 and Comparative Example 1 are shown in Fig. 3, the results of Example 2 and Comparative Example 2 in Fig. 4, and the results of Example 3 and Comparative Example 3 in Fig. 5, the results of Example 5 and Comparative Example 5 in Fig. 6, respectively. The results of Example 4 and Comparative Example 4 are not shown, but after 18 hours and 24 hours, the cumulative amount of the drug permeated [Q] of the patch obtained in Example 4 was about twice as much as the cumulative amount of the drug permeated [Q] of the patch obtained in Comparative Example 4.

In Example 3 and Comparative Example 3, three samples for each patch were prepared in the same conditions and the cumulative amount of the drug permeated [Q] was calculated in the same way as above for each patch and the standard deviation of the cumulative amount of the drug permeated [Q] when taking each sample liquid was obtained. It was observed that the skin permeation of the obtained patch was more stable with a smaller value of the standard deviation. The standard deviation of the cumulative amount of the drug permeated [Q] when taking each sample liquids is shown in Table 3.

[Table 3]

| Time (hour) | Standard Deviation ($\mu g/cm^2$) | |
|---|---|---|
| | Example 3 (Sodium Diacetate) | Comparative Example 3 (Acetic Acid + Sodium Acetate) |
| 0 | 0 | 0 |
| 6 | 8.9 | 11.4 |
| 12 | 19.7 | 39.7 |
| 18 | 26.5 | 57.3 |
| 24 | 30.0 | 65.5 |

Furthermore, the X-ray diffraction angles were measured for the patches obtained in Examples 3 to 4 and Comparative Examples 3 to 4, respectively. The spectra of respective patches at an X-ray diffraction angle of 2θ° are shown in Figs. 7 to 10. Figs. 7, 8, 9, and 10 are graphs showing the spectra at an X-ray diffraction angle of 2θ° of respective patches obtained in Example 3, Comparative Example 3, Example 4, and Comparative Example 4, respectively. A peak having high intensity from sodium diacetate was observed (near 22.4°) in the results shown in Figs. 7 and 9, whereas the same peak having only low intensity was observed in the results shown in Figs. 8 and 10. On the other hand, a peak having high intensity from sodium acetate was observed (near 8.7°) in the results shown in Figs. 8 and

10, whereas the same peak was not observed in the results shown in Figs. 7 and 9.

In addition, the storage stability evaluation test was performed on the patches obtained in Example 1 and Comparative Example 1. The results are shown in Fig. 11. According to visual observation of the appearance of each patch, the patch obtained in Comparative Example 1 was discolored brown after one-week storage, whereas the patch obtained in Example 1 was observed to have no particular change in color tone.

As apparent from the results of the above skin permeation test, it was observed that the patches obtained by the production method of the present invention had significantly high skin permeation of the drug as compared with patches containing oleic acid, acetic acid, and sodium acetate which were conventionally known as compounds for improving the skin permeation of a drug. As apparent from the results shown in Figs. 7 to 10, it was observed that the patches obtained by the production method of the present invention contained a large amount of sodium diacetate and it was observed that the skin permeation of the drug in the patches was improved by using the sodium diacetate according the present invention.

On the other hand, as apparent from the results shown in Figs. 7 to 10, it was observed that although a few complexes similar to sodium diacetate were formed by acetic acid and sodium acetate in the patches (Comparative Examples 3 to 4) obtained using acetic acid and sodium acetate, a large amount of sodium acetate remained and accordingly a large amount of acetic acid that was not able to form a complex with sodium acetate also remained. Furthermore, as apparent from the results shown in Fig. 3, it was observed that the patch (Comparative Example 3) obtained using acetic acid and sodium acetate had variation in skin permeation of the drug among pharmaceutical preparations and the variation increased with time, whereas the patches obtained by the production method of the present invention had less variation among pharmaceutical preparations.

As apparent from the results shown in Figs. 3 and 11, it was observed that the patch (Comparative Example 1) using the free type drug had relatively high skin permeation of the drug but was inferior in storage stability. On the other hand, it was observed that the patch obtained in Example 1 was excellent in both skin permeation of the drug and storage stability.

[Industrial Applicability

As described above, the present invention can provide the method for producing the patch, which can easily give the patch excellent in skin permeation of the drug and can reduce variation in skin permeation of the drug for each pharmaceutical preparation, and the patch obtained by the production method.

## Claims

1. A method for producing a patch including a support layer and an adhesive layer, comprising the step of forming the adhesive layer with use of an adhesive layer composition obtained by mixing an alkali metal diacetate, a drug, and a nonaqueous adhesive base such that the molar ratio between the drug and the alkali metal diacetate (the number of moles of the drug: the number of moles of the alkali metal diacetate) is from 1:0.5 to 1:15,
wherein
the drug is at least one selected from the group consisting of fumaric acid addition salts of a basic drug, maleic acid addition salts of a basic drug, citric acid addition salts of a basic drug, and hydrochloric acid addition salts of a basic drug,
the basic drug is at least one selected from the group consisting of emedastine, setiptiline, and oxybutynin, and
the nonaqueous adhesive base is at least one selected from the group consisting of a styrene-isoprene-styrene block copolymer, a (meth)acrylate (co)polymer, polyisobutylene, and a silicone polymer.

2. The method for producing a patch according to claim 1, wherein the alkali metal diacetate is sodium diacetate.

## Patentansprüche

1. Verfahren zur Herstellung eines Pflasters mit einer Trägerschicht und einer Klebeschicht, umfassend den Schritt des Bildens der Klebeschicht unter Verwendung einer Klebeschichtzusammensetzung, die durch Mischen eines Alkalimetalldiacetats, eines Medikaments und einer nichtwässrigen Klebegrundmasse erhalten wird, so dass das Molverhältnis zwischen dem Medikament und dem Alkalimetalldiacetat (die Anzahl der Mole des Medikaments : die Anzahl der Mole des Alkalimetalldiacetats) von 1:0,5 bis 1:15 beträgt,
wobei
das Medikament mindestens eines ist ausgewählt aus der Gruppe bestehend aus Fumarsäure-Additionssalzen

eines basischen Medikaments, Maleinsäure-Additionssalzen eines basischen Medikaments, Zitronensäure-Additionssalzen eines basischen Medikaments und Salzsäure-Additionssalzen eines basischen Medikaments,
das basische Medikament mindestens eines ist ausgewählt aus der Gruppe bestehend aus Emedastin, Setiptilin und Oxybutynin, und
die nichtwässrige Klebegrundmasse mindestens eine ist ausgewählt aus der Gruppe bestehend aus einem Styrol-Isopren-Styrol-Blockcopolymer, einem (Meth)acrylat(co)polymer, Polyisobutylen und einem Silikonpolymer.

2. Verfahren zur Herstellung eines Pflasters nach Anspruch 1, worin das Alkalimetalldiacetat Natriumdiacetat ist.

## Revendications

1. Procédé de production d'un timbre comprenant une couche formant support et une couche adhésive, comprenant l'étape de formation de la couche adhésive avec l'utilisation d'une composition de couche adhésive obtenue par mélange d'un diacétate de métal alcalin, d'un médicament, et d'une base adhésive non aqueuse de sorte que le rapport molaire entre le médicament et le diacétate de métal alcalin (le nombre de moles du médicament:le nombre de moles du diacétate de métal alcalin) est de 1:0,5 à 1:15,
où
le médicament est au moins l'un sélectionné dans le groupe constitué des sels d'addition d'acide fumarique d'un médicament basique, des sels d'addition d'acide maléique d'un médicament basique, des sels d'addition d'acide citrique d'un médicament basique, et des sels d'addition d'acide chlorhydrique d'un médicament basique,
le médicament basique étant au moins l'un sélectionné dans le groupe constitué de l'émédastine, de la setiptiline, et de l'oxybutynine, et
la base adhésive non aqueuse étant au moins l'une sélectionnée dans le groupe constitué d'un copolymère séquencé de styrène-isoprène-styrène, d'un (co)polymère (méth)acrylate, de polyisobutylène, et d'un polymère de silicone.

2. Procédé de production d'un timbre selon la revendication 1, le diacétate de métal alcalin étant le diacétate de sodium.

Fig. 1

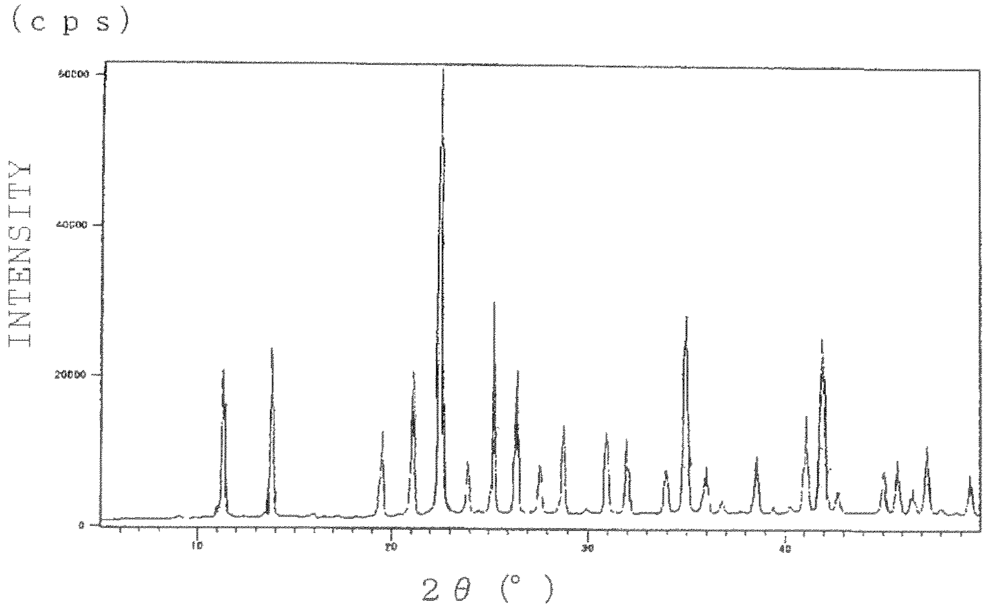
(c p s)
INTENSITY
60000
40000
20000
0
10
20
30
40
2θ (° )

Fig. 2

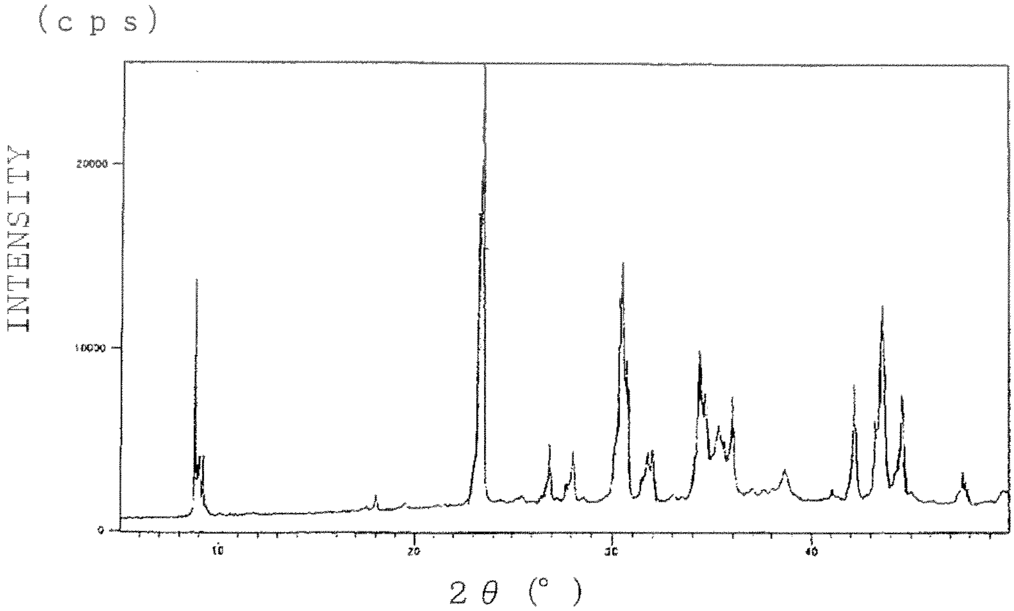
(c p s)
INTENSITY
20000
10000
0
10
20
30
40
2θ (° )

Fig. 3

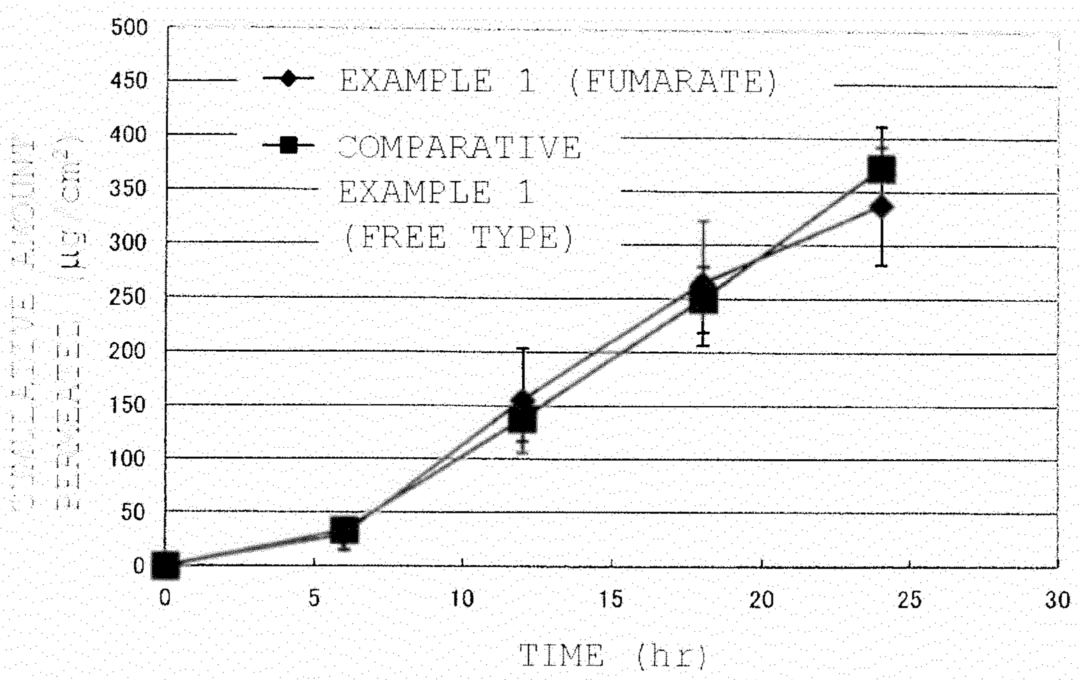
EXAMPLE 1 (FUMARATE)
COMPARATIVE EXAMPLE 1 (FREE TYPE)
(μg/cm²)
0
50
100
150
200
250
300
350
400
450
500
0
5
10
15
20
25
30
TIME (hr)

Fig. 4

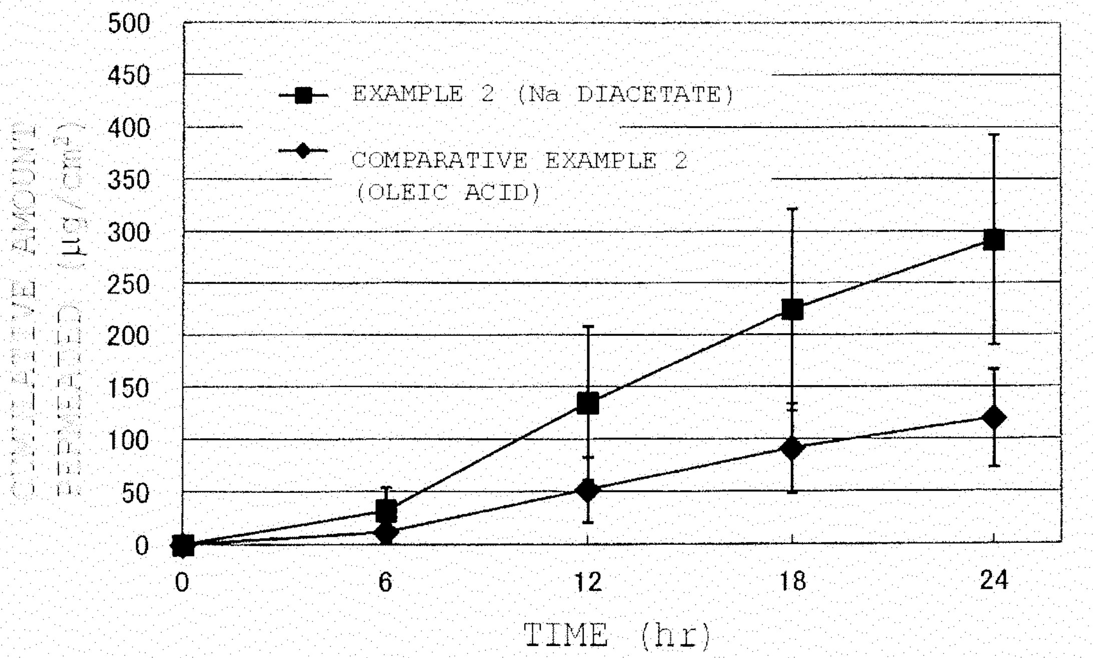
EXAMPLE 2 (Na DIACETATE)
COMPARATIVE EXAMPLE 2 (OLEIC ACID)
CUMULATIVE AMOUNT PERMEATED (μg/cm²)
0
50
100
150
200
250
300
350
400
450
500
0
6
12
18
24
TIME (hr)

Fig. 5

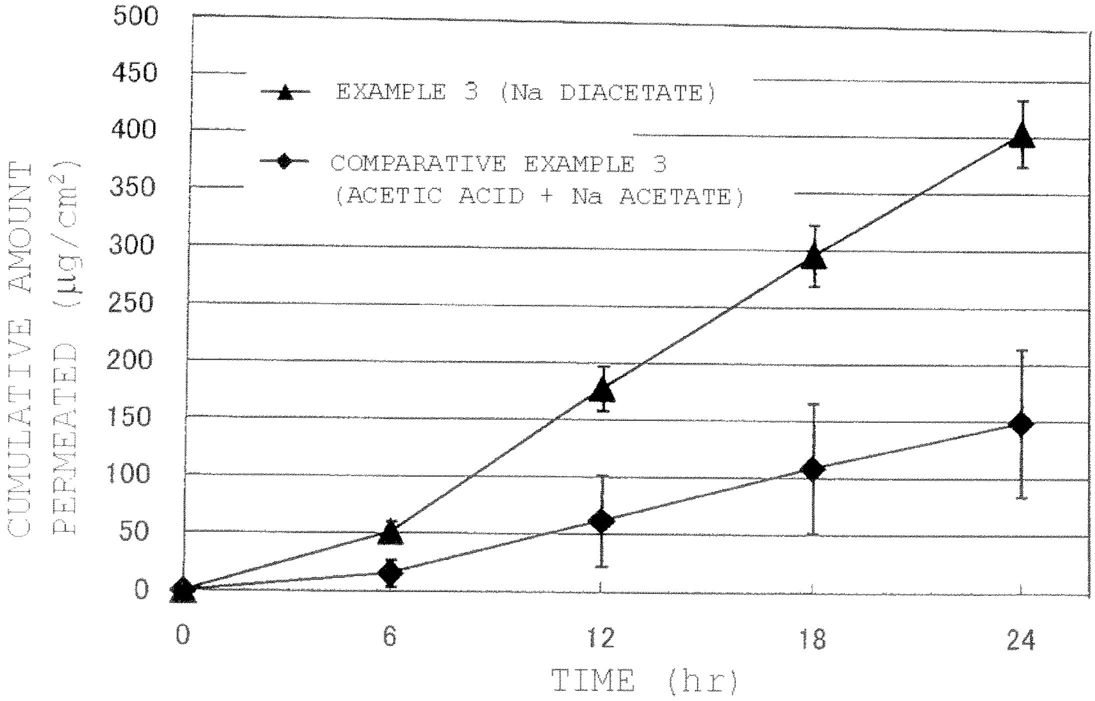
500
450
400
350
300
250
200
150
100
50
0
0
6
12
18
24
TIME (hr)
CUMULATIVE AMOUNT PERMEATED (μg/cm²)
EXAMPLE 3 (Na DIACETATE)
COMPARATIVE EXAMPLE 3 (ACETIC ACID + Na ACETATE)

Fig. 6

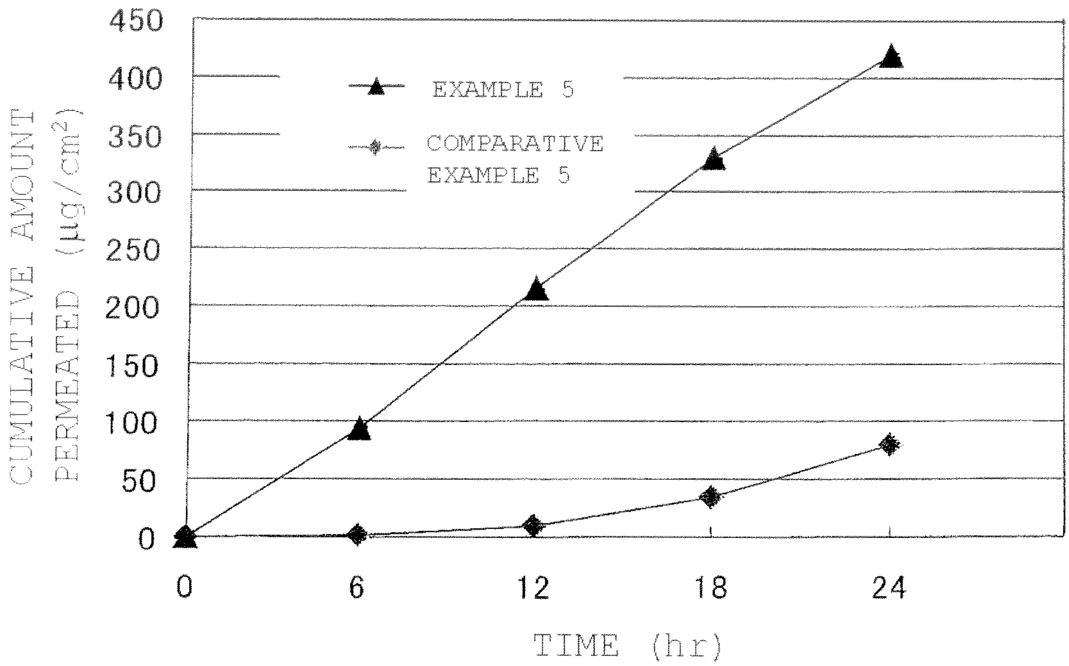
450
400
350
300
250
200
150
100
50
0
0
6
12
18
24
TIME (hr)
CUMULATIVE AMOUNT PERMEATED (μg/cm²)
EXAMPLE 5
COMPARATIVE EXAMPLE 5

Fig. 7

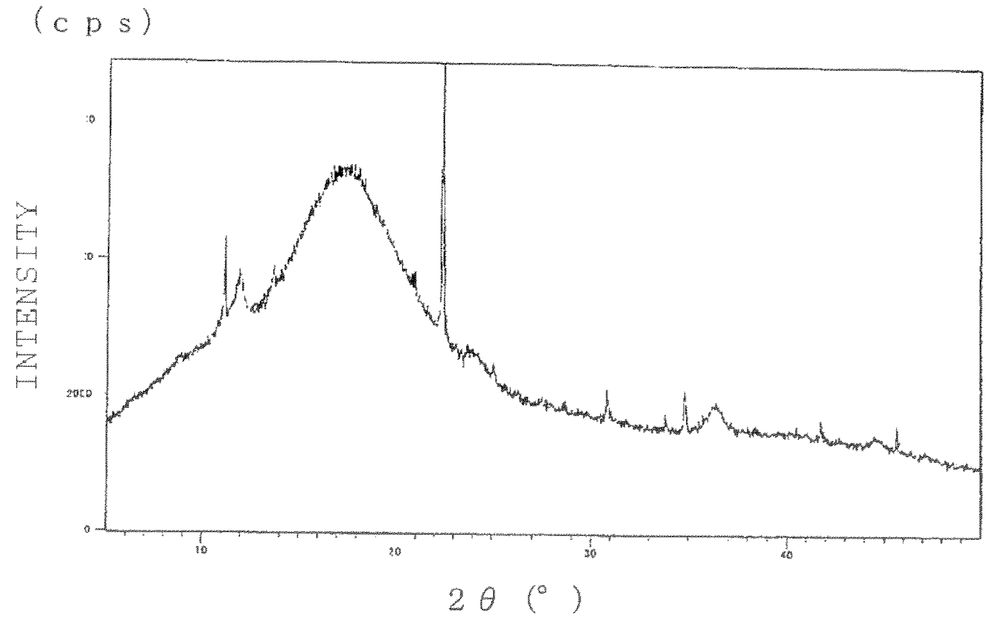
(cps)
INTENSITY
2000
0
10
20
30
40
2θ (°)

Fig. 8

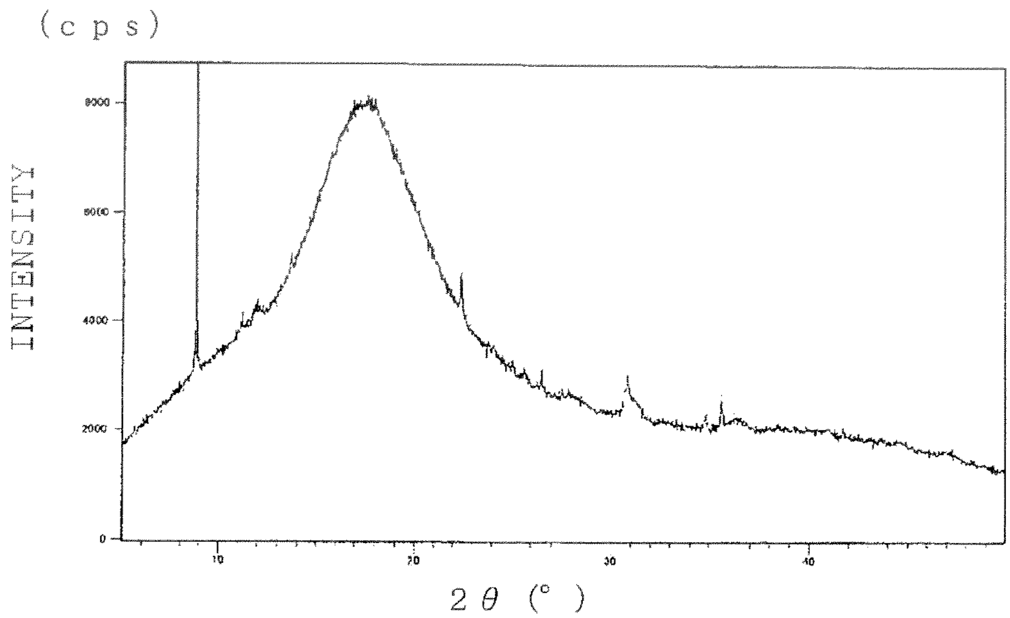
(cps)
INTENSITY
8000
6000
4000
2000
0
10
20
30
40
2θ (°)

Fig. 9

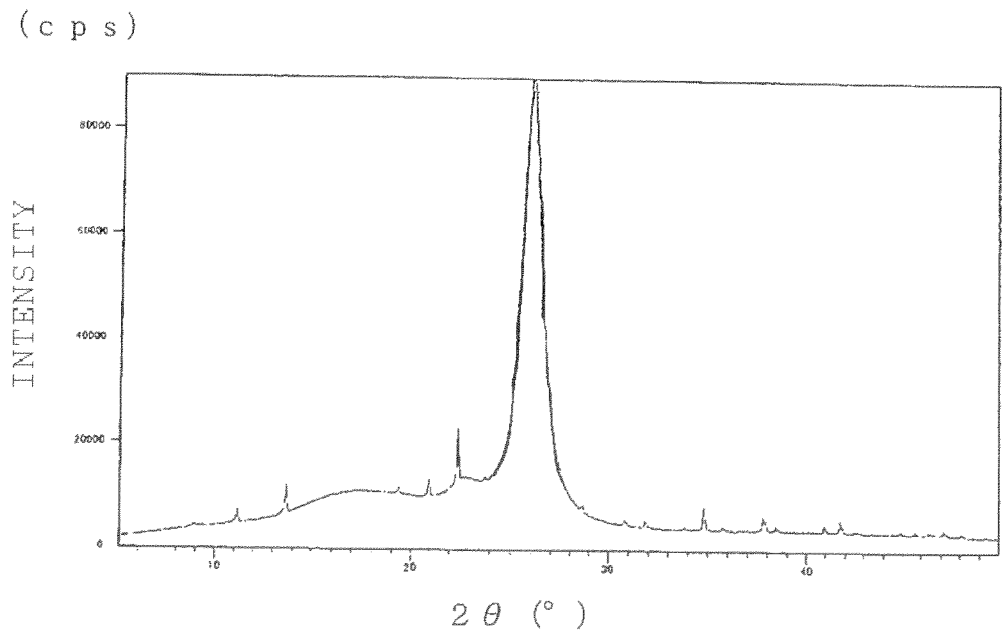
(cps)
INTENSITY
80000
60000
40000
20000
0
10
20
30
40
2θ (°)

Fig. 10

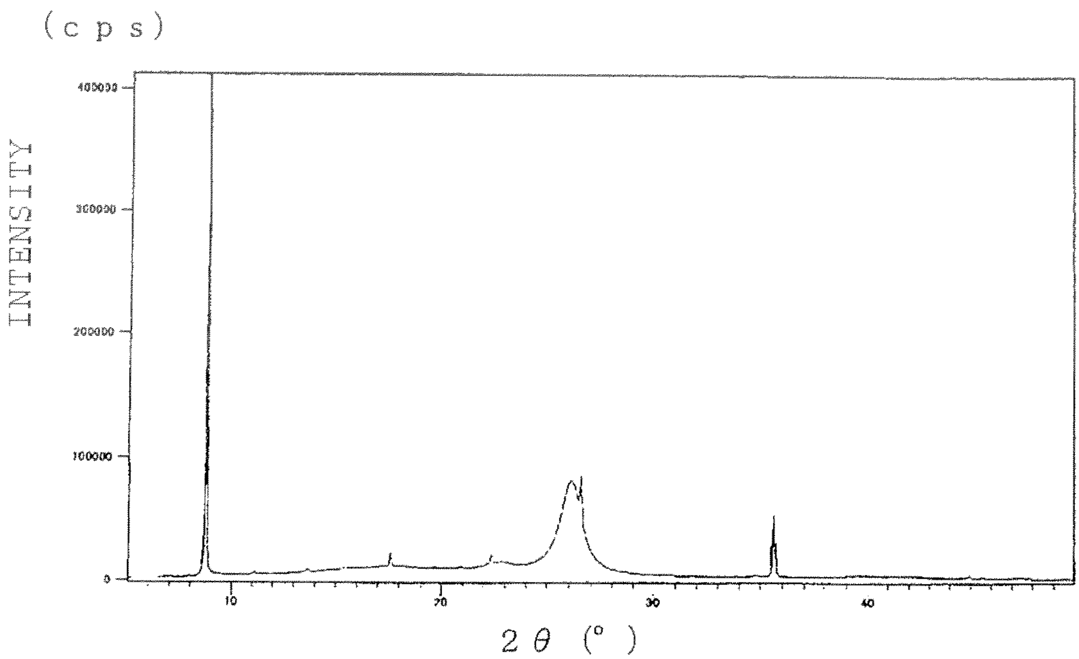
(cps)
INTENSITY
400000
300000
200000
100000
0
10
20
30
40
2θ (°)

Fig. 11

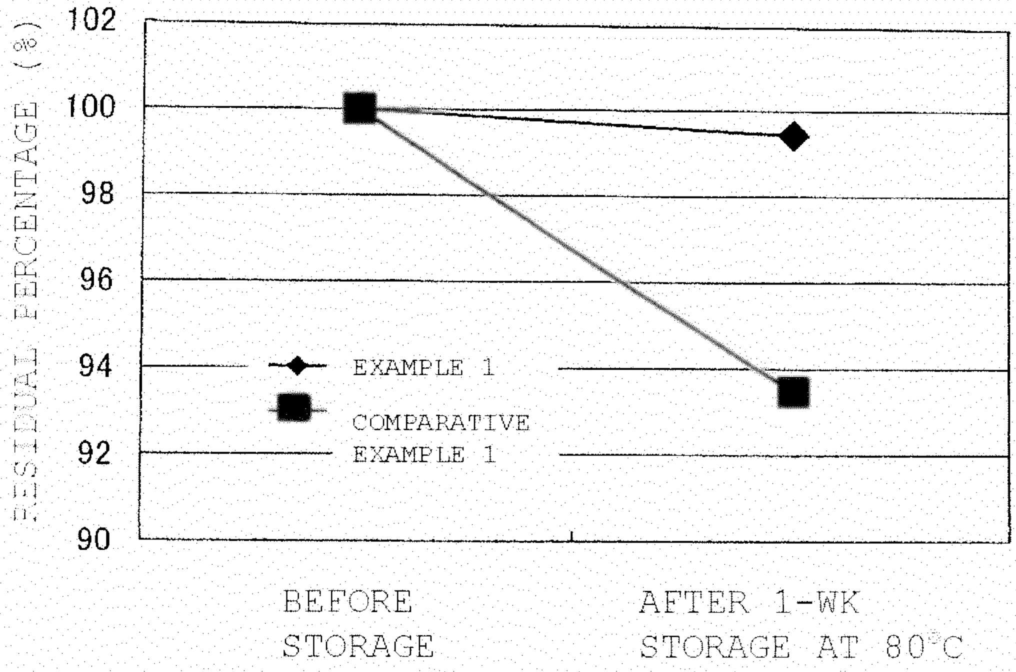
RESIDUAL PERCENTAGE (%)
102
100
98
96
94
92
90
EXAMPLE 1
COMPARATIVE
EXAMPLE 1
BEFORE
STORAGE
AFTER 1-WK
STORAGE AT 80°C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP HEI11302161 B
- WO 0107018 A
- WO 0061120 A
- WO 2005115355 A
- WO 02069942 A
- JP 2004500360 A
- WO 2009110351 A
- JP HEI383924 B
- JP HEI733665 B
- JP HEI8193030 B
- JP 2255612 A
- JP HEI3197420 B
- US 20080226697 A
- US 20090004255 A
- US 20060110434 A
- JP HEI2255612 B